# EUROPEAN PATENT APPLICATION

(11) **EP 3 804 694 A1**
(43) Date of publication of application: **14.04.2021**
(21) Application number: 19815641.6
(22) Date of filing: 05.06.2019
(51) Int. Cl.: A61K 8/9794, A61Q 17/00, A61Q 19/02, A61Q 19/08

(54) **COMPOSITION CONTAINING DENDROBIUM NOBILE AND METHOD OF USING SAME**

(30) Priority: 06.06.2018 CN 201810573017
(71) Applicant: ACCESS BUSINESS GROUP INTERNATIONAL LLC, Ada, Michigan 49355 (US)
(72) Inventor: HUANG, Manlian, Shanghai 201203 (CN); SONG, Xianli, Shanghai 201203 (CN); HUANG, Juan, Shanghai 201203 (CN); LI, Bo, Shanghai 201203 (CN)
(74) Representative: Boult Wade Tennant LLP
(86) International application number: PCT/IB2019/054649
(87) International publication number: WO 2019/234635

(57) **Abstract**

Compositions, packaging systems and methods for protecting human skin and/or hair against air pollution-induced damage including *Dendrobium,* e.g., *Dendrobium nobile* are described.

## Description

### BACKGROUND

Compositions for preventing, treating and/or curing human skin from an air pollution-induced damage and methods of using the compositions are described.

The human skin is the outer covering of the body. In humans, it is the largest organ of the integumentary system. The skin has multiple layers of ectodermal tissue and guards the underlying muscles, bones, ligaments and internal organs.

The two major layers of the skin include the stratum corneum, or epidermis, and the dermis layers. The epidermis is the top or outer layer of the skin and its primary function is to provide a protective covering and retard evaporative water loss from the aqueous interior. The epidermis protects against mechanical insults, the ingress of foreign chemicals and pollutants, and assaults by microorganisms. It also provides the first defense against ultraviolet light. The dermis lies under the epidermis and makes up 90 percent of the skin's thickness. The dermis contains a dense meshwork of collagen and elastin, providing strength and elasticity to the skin. Fibroblasts constitute the main cell type present in the dermis and are responsible for synthesis and secretion of dermal matrix components, including collagen, elastin, and glycosaminoglycans (such as hyaluronic acid). Whereas collagen provides strength to the skin and elastin its elasticity, glycosaminoglycans serve to keep the skin moist and plump.

To stay healthy, the skin must cope with changing environmental conditions and air pollution, while simultaneously repairing any damage to the skin. Environmental factors and air pollution play a chief role in aging, wrinkles, skin discolorations, degenerative skin conditions, and skin diseases, such as skin cancer.

Air pollution occurs when harmful substances including particulates and biological molecules are introduced into Earth's atmosphere. It may cause diseases, allergies or death of humans; it may also cause harm to other living organisms such as animals and food crops, and may damage the natural or built environment. Human activity and natural processes can both generate air pollution. Exposure to air pollution can have a negative effect on skin causing skin aging, formation of wrinkles and inflammations, and perhaps, even skin cancer. The exposure of human skin to repeated air pollution was shown in epidemiological studies to support extrinsic skin aging associated with pigment spots and wrinkles (Vierkoetter et al., J Invest Dermatol, 130 (12):2719-26 (2010)).

Exposure to sunlight and UV radiation are also major factors resulting in skin damage, accounting for 90% of the symptoms of premature aging. Importantly, exposure to oxygen, sunlight, and other environmental or lifestyle stresses induces the formation of free radicals. Free radicals can cause wrinkles by activating metalloproteases, such as collagenases, that are responsible for breaking down the skin's connective tissues (collagen and elastin). The result is premature aging. Free-radical damage can also cause a reduction in the thickness of the dermal layer. This can cause the skin to slacken. The slackening of the skin is the first and most visible sign of aging and a cause of wrinkles and lines.

In view of the many detrimental effects impacting the skin, there is a demand for compositions and methods for not only improving the appearance and condition of skin but also ones that simultaneously fight the negative consequences that air pollutants provide to human skin and protect skin cells against air-pollution induced skin damage. Advantageously, these compositions and methods would, optionally, also fight the negative consequences of exposure of the skin to sunlight and UV radiation and/or other environmental conditions.

There also remains a need for effective topically applied cosmetic compositions that provide anti-pollution benefits to the skin, hair and/or nails using natural ingredients as active components. Unnatural, chemically synthesized products may be perceived as being environmentally or personally unsafe. In contrast, natural products are perceived as pure, mild, and superior to chemically synthesized products. Natural based products extracted from plants or herbs are believed to contain antioxidant/free-radical scavenging agents that can neutralize the effects of free-radical damage. Additionally, natural-based products can contain agents that stimulate the synthesis and restoration of damaged connective tissue structures in the dermis and barrier function in the epidermis.

However, delivering a cosmetic benefit from "natural" sources, such as plants or herbs, is not trivial. Deriving a real benefit from such sources requires identification of specific plant/herbal extracts or ingredients, their minimum active concentrations, and their additive or synergistic activities in combination with other ingredients to impart anti-pollution and/or skin improvement benefits.

Provided herein are compositions and methods for preventing, treating and/or curing human skin and/or hair from air pollution-induced and/or air pollution-inducible skin and/or hair damage, optionally having additional skin benefits.

### SUMMARY

One embodiment relates to a cosmetic composition comprising an isolated ingredient or an extract of *Dendrobium nobile,* in a working amount sufficient for at least one of: reducing or preventing air pollution-induced or air pollution-inducible skin and/or hair damage; reducing or preventing air pollution-induced oxidative stress; reducing or preventing air pollution-induced decrease in cell vitality; reducing or preventing air pollution-induced increase in IL-6; or reducing or preventing air pollution-induced cell damage; and a cosmetically acceptable carrier or excipient. The skin damage is skin aging, lines, fine lines, wrinkles, crow's feet, dark eye circles, blemishes, age spots, dark spots, stretch marks, skin cancer, skin inflammation, skin discoloration, skin hyperpigmentation, or combinations thereof. The composition may be a personal care composition, a skin care composition, a hair care composition, an anti-aging care composition, a sun care composition, or a combination thereof. The composition may be in a form of an aerosol, a cream, a emulsion, a solid, a liquid, a dispersion, a foam, a gel, a lotion, a mousse, an ointment, a powder, a patch, a pomade, a solution, a pump a spray, a stick, am oil, a towelette, or combinations thereof. The ingredient or the extract of *Dendrobium nobile* may be present in an amount of from about 0.0001% to about 5% by weight of the total composition. The ingredient or the extract of *Dendrobium nobile* may be present in an amount of from about 0.01% to about 0.1% by weight of the total composition. The cosmetically acceptable carrier or excipient may be selected from the group consisting of water, a glycerin, a C 1-C4 alcohol, a fatty alcohol, a fatty ether, a fatty ester, a polyol, a glycol, a vegetable oil, a mineral oil, a liposome, a laminar lipid material, a silicone oil, or combinations thereof.

Another embodiment relates to a medicament comprising an ingredient or an extract of *Dendrobium nobile* and a pharmaceutically acceptable carrier or excipient, wherein the medicament is for preventing, treating and/or curing human skin and/or hair from at least one of: air pollution-induced or air pollution-inducible skin and/or hair damage; air pollution-induced oxidative stress; air pollution-induced decrease in cell vitality; air pollution-induced increase in IL-6; or air pollution-induced cell damage. The skin damage may be skin aging, lines, fine lines, wrinkles, crow's feet, dark eye circles, blemishes, age spots, dark spots, stretch marks, skin cancer, skin inflammation, skin discoloration, skin hyperpigmentation, skin barrier function damage, or combinations thereof. The ingredient or the extract of *Dendrobium nobile* may be present in an amount of from about 0.000 1% to about 5% by weight of the total composition. Alternatively, the ingredient or the extract of *Dendrobium nobile* may be present in an amount of from about 0.01 % to about 0.1% by weight of the total composition.

Yet further embodiment relates to a packaging system comprising one or more containers collectively containing the described composition and instructions for applying the composition from the one or more containers. The packaging system can include at least one of: (a) one or more containers collectively containing the composition described herein; or (b) instructions for applying the composition from the one or more containers.

Another embodiment relates to a method for protecting human skin and/or hair against air pollution-induced damage comprising topically applying the described composition to the skin and/or hair. An ingredient or an extract of *Dendrobium nobile* is applied to human skin and/or hair in an effective amount of about 0.01 to about 5 mg/cm². Alternatively, an ingredient or an extract of *Dendrobium nobile* is applied to human skin and/or hair in an effective amount of about 0.05 to about 5 mg/cm²

Yet another embodiment relates to the use of an ingredient or an extract of *Dendrobium nobile* as an anti-pollution agent for protecting human skin and/or hair. The anti-pollution is directed to protection and/or inhibition and/or reduction of disorders and dysfunctions of human skin and/or hair.

### BRIEF DESCRIPTION OF THE DRAWINGS

Figure 1 shows a bar graph demonstrating of PM2.5 toxicity test.
Figure 2 shows a bar graph of *Dendrobium nobile* extract toxicity test at 4 hours and 24 hours post treatment.
Figure 3 shows a bar graph demonstrating that *Dendrobium nobile* extract can effectively counter the reactive oxygen species (ROS) level increased by PM2.5 without causing oxidative stress of Hacat cells.
Figure 4 depicts a bar graph showing mitochondrial membrane potential (MMP) test results; PM2.5 significantly decreases the MMP level, while *Dendrobium nobile* extract protects cell vitality.
Figure 5 depicts a bar graph showing IL-6 level as induced by various test samples; PM2.5 significantly increases IL-6 levels, while *Dendrobium nobile* extract significantly decreases the IL-6 level (by 41.3%) as compared with PM2.5 treatment group.
Figure 6 depicts a bar graph demonstrating that PM2.5 can increase cell damage rate, while *Dendrobium nobile* extract does not damage the cell. Instead, it can prevent the cells from the damage caused by treatment with PM2.5.

### DETAILED DESCRIPTION

It is to be understood that this invention is not limited to the particular compositions, methodology, or protocols described herein. Further, unless defined otherwise, all technical and scientific terms used herein have the same meaning as commonly understood to one of ordinary skill in the art to which this invention belongs. It is also to be understood that the terminology used herein is for the purpose of describing particular embodiments only, and is not intended to limit the scope of the present invention, which will be limited only by the claims.

The present invention is based on the surprising discovery that a plant ingredient and/or a plant extract of *Dendrobium nobile* reduces and/or prevents air pollution-induced or air pollution-inducible skin and/or hair damage, reduces and/or prevents air pollution-induced oxidative stress, reduces and/or prevents air pollution-induced decrease in cell vitality, reduces and/or prevents air pollution-induced increase in IL-6, and/or reduces or prevents air pollution-induced cell damage. The ingredient or extract may be from any species members of genus *Dendrobium* groups; preferably, the ingredient or extract may be an ingredient or extract of *Dendrobium nobile.*

In view of this, compositions that include an isolated plant ingredient and/or plant extract of *Dendrobium,* e.g., *Dendrobium nobile,* in a sufficient amount to achieve prevention, treatment and/or cure to human skin and/or hair from at least one of: air pollution-induced or air pollution-inducible skin and/or hair damage, air pollution-induced oxidative stress, air pollution-induced decrease in cell vitality, air pollution-induced increase in IL-6; and/or air pollution-induced cell damage, and the use of these compositions are described.

The term "composition" refers to a product that treats, improves, promotes, increases, manages, controls, maintains, optimizes, modifies, reduces, inhibits, or prevents a particular condition associated with a natural state, biological process or disease or disorder. For example, a composition described herein improves at least one of: pollution-induced or air pollution-inducible skin and/or hair damage, air pollution-induced oxidative stress, air pollution-induced decrease in cell vitality, air pollution-induced increase in IL-6; and/or air pollution-induced cell damage, and the like in a subject. The term composition includes, but is not limited to, pharmaceutical (i.e., drug), over-the counter (OTC), cosmetic, food, food ingredient or dietary supplement compositions that include an effective amount of an extract, at least one component thereof, or a mixture thereof.

Exemplary forms of compositions described herein include an aerosol, a cream, a emulsion, a solid, a liquid, a dispersion, a foam, a liniment foam, a gel, a lotion, a mousse, an ointment, a pack or powder, an emulsion, a patch, a pomade, a solution, a pump a spray, a stick, am oil, a towelette, tablets, plasters, granules, and/or combinations thereof.

The term "air pollution" refers to any contamination of air, of natural origin or man-made, by smoke and/or harmful gases (e.g., the exhausts of traffic and the exhausts of industry), mainly oxides of carbon, sulfur, and nitrogen, any released particles, or liquid droplets. For example, any particles released by abrasion of rubber wheels may be considered air-pollutants. These air pollutants may have bound polycyclic aromatic hydrocarbons (PAH) but also include others chemicals. Also, carbon black particles released by printers may be considered air pollutants in an indoor environment. Indoor cooking, for example with coal or firewood may also generate air pollutants.

An "air pollutant" is a substance in the air that can have adverse effects on humans and the ecosystem and contributes to air pollution. The substance can be solid particles, liquid droplets, or gases. A pollutant can be of natural origin or man-made. Pollutants are classified as primary or secondary. Primary pollutants are usually produced from a process, such as ash from a volcanic eruption. Other examples include carbon monoxide gas from motor vehicle exhaust, or the sulfur dioxide released from factories. Secondary pollutants are not emitted directly. Rather, they form in the air when primary pollutants react or interact. Ground level ozone is a prominent example of a secondary pollutant. Some pollutants may be both primary and secondary: they are both emitted directly and formed from other primary pollutants.

Exemplary primary air pollutants include: carbon dioxide (CO₂) (emitted by, e.g., burning of fossil fuels); sulfur oxides (SOₓ), e.g., sulfur dioxide (SO₂) produced by volcanoes and in various industrial processes (e.g., coal and petroleum often contain sulfur compounds, and their combustion generates sulfur dioxide); nitrogen oxides (NOₓ), e.g., nitrogen dioxide (NO₂), which is expelled from high temperature combustion; carbon monoxide (CO), which is a colorless, odorless, toxic yet non-irritating gas and is a product of combustion of fuel such as natural gas, coal or wood and by vehicular exhaust; volatile organic compounds (VOC), a well-known outdoor air pollutant, e.g., either methane (CH₄) or non-methane (NMVOCs) (the aromatic NMVOCs benzene, toluene and xylene are suspected carcinogens; 1,3-butadiene is another dangerous compound often associated with industrial use); particulates, alternatively referred to as particulate matter (PM), atmospheric particulate matter, or fine particles, are tiny particles of solid or liquid suspended in a gas; aerosol ("combined particles and gas") - some particulates occur naturally, originating from volcanoes, dust storms, forest and grassland fires, living vegetation, and sea spray, some from human activities, such as the burning of fossil fuels in vehicles, power plants and various industrial processes also generate significant amounts of aerosols; toxic metals, such as lead and mercury, especially their compounds; chlorofluorocarbons (CFCs) - emitted from products are currently banned from use, these are gases which are released from air conditioners, refrigerators, aerosol sprays, etc. - on release into the air, CFCs rise to the stratosphere, here they come in contact with other gases and damage the ozone layer and allow harmful ultraviolet rays to reach the earth's surface leading to, e.g., skin cancer or eye disease; and ammonia (NH₃) - emitted from agricultural processes - is normally encountered as a gas that can react with oxides of nitrogen and sulfur to form secondary particles.

Exemplary secondary air pollutants include particulates created from gaseous primary pollutants and compounds in photochemical smog. Smog is a kind of air pollution. Classic smog results from large amounts of coal burning in an area caused by a mixture of smoke and sulfur dioxide. Modern smog does not usually come from coal but from vehicular and industrial emissions that are acted on in the atmosphere by ultraviolet light from the sun to form secondary pollutants that also combine with the primary emissions to form photochemical smog. Ground level ozone (O₃) formed from NOₓ and VOCs. Ozone (O₃) is a key constituent of the troposphere. It is also an important constituent of certain regions of the stratosphere commonly known as the Ozone layer. Photochemical and chemical reactions involving it drive many of the chemical processes that occur in the atmosphere by day and by night. At abnormally high concentrations brought about by human activities (largely the combustion of fossil fuel), it is a pollutant, and a constituent of smog. Peroxyacetyl nitrate (C₂H₃NO₅), similarly formed from NOₓ and VOCs, is another example of a secondary air pollutant.

The term "air pollution-induced skin and/or hair damage" refers to any skin and/or hair damage, disease, and/or condition brought about by a short-term and/or long-term exposure to air pollution. The air pollution-induced skin and/or hair damage may manifest as, e.g., skin aging; formation of wrinkles and inflammations; formation of lines (e.g., fine lines), wrinkles, crow's feet; formation of dark eye circles; formation of blemishes, acne, age spots, dark spots, stretch marks; formation of cancer (including skin cancer); skin discoloration, skin hyperpigmentation, or combinations thereof. Additionally, the air pollution-induced skin and/or hair damage may manifest as psoriasis, eczema, seborrhea, dermatitis, sunburn, estrogen imbalance, yellowing, freckles, skin atrophy, skin breakout, skin fragility, dryness, tactile roughness, chapping, sagginess, thinning, hyperplasia, fibrosis, enlarged pores, cellulite formation, bruising, apoptosis, cellular differentiation, cellular de-differentiation, viral infections, fungal infections, bacterial infections, spider veins (telangectasia), hirsutism, rosacea, pruritis, calluses, warts, corns, or combinations thereof.

The term "air pollution-inducible skin and/or hair damage" refers to any skin and/or hair damage, disease, and/or condition that can be formed in response to the presence of air pollutants.

As used herein, the term "extract" or "botanical extract" refers to a solid, viscid, or liquid substance or preparation that includes an isolated active ingredient(s) of a substance of plant, such as *Dendrobium* (e.g., *Dendrobium nobile).* The ingredient or extract may be from any species members of genus *Dendrobium.* The term "extract" is intended to include not only a crude extract produced from *Dendrobium* (e.g., *Dendrobium nobile),* and by use of a solvent selected from among water, lower alcohols of I to 4 carbon atoms, such as methanol, ethanol, butanol, etc., ethylene, acetone, hexane, ether, chloroform, ethylacetate, butylacetate, dichloromethane, N,N-dimethylformamide (DMF), dimethylsulfoxide (DMSO), 1,3-butylene glycol, propylene glycol and a combination thereof, but also a fraction of the crude extract in such a solvent. So long as it assures the extraction and preservation of the active ingredient(s), any extraction method may be employed. On examples of botanical extracts includes an extract from *Dendrobium nobile* plant.

As used herein, the terms "amount sufficient," "effective amount" or "therapeutically effective amount" of a pure compound, composition, extract, extract mixture, component of the extract, and/or active agent or ingredient, or a combination thereof refers to an amount effective at dosages and for periods of time sufficient to achieve a desired result. For example, the "amount sufficient," "effective amount" or "therapeutically effective amount" refer to that amount of a pure compound, composition, extract, botanical extract, extract mixture, botanical extract mixture, component of the extract, and/or active agent or ingredient, or a combination thereof which, when administered to a subject (e.g., mammal, such as a human), is sufficient to effect prevention, treatment, and or cure of, e.g., pollution-induced or air pollution-inducible skin and/or hair damage, air pollution-induced oxidative stress, air pollution-induced decrease in cell vitality, air pollution-induced increase in IL-6; and/or air pollution-induced cell damage, and the like in a subject. The amount of a composition, extract, botanical extract, extract mixture, botanical extract mixture, component of the extract, and/or active agent or ingredient of this disclosure that constitutes an "effective amount" or "therapeutically effective treatment" will vary depending on the active agent or the compound, the condition being treated and its severity, the manner of administration, the duration of treatment, or the age of the subject to be treated, but can be determined routinely by one of ordinary skill in the art having regard to his own knowledge and to this disclosure.

The term "protecting, treating and/or curing human skin and/or hair against air pollution-induced damage" designates preventing, arresting, reversing, ameliorating, diminishing, and/or reducing air pollution-induced damage, especially to human skin and/or hair. Representative signs of air pollution-induced damage to the skin and hair include, but are not limited to, skin aging, lines, fine lines, wrinkles, crow's feet, dark eye circles, blemishes, age spots, dark spots, stretch marks, cancer (including skin cancer), skin inflammation, skin discoloration, skin hyperpigmentation, or combinations thereof. Other signs of air pollution-induced damage to the skin and hair include psoriasis, eczema, seborrhea, dermatitis, sunburn, estrogen imbalance, yellowing, freckles, skin atrophy, skin breakout, skin fragility, dryness, tactile roughness, chapping, sagginess, thinning, hyperplasia, fibrosis, enlarged pores, cellulite formation, bruising, acne formation, apoptosis, cellular differentiation, cellular de-differentiation, prevention of tumor induction or tumor progression, viral infections, fungal infections, bacterial infections, spider veins (telangectasia), hirsutism, rosacea, pruritis, calluses, warts, corns, or combinations thereof.

The terms "cosmetically acceptable" and "pharmaceutically acceptable" mean those drugs, medicaments, ointments, extracts or inert ingredients, which are suitable for use in contact with the tissues of humans and lower animals without undue toxicity, incompatibility, instability, irritation, and the like, commensurate with a reasonable benefit/risk ratio.

The terms "administering" and "applying" can use interchangeably and are defined as providing a composition to a subject via a route known in the art, including but not limited to topical, oral, intravenous, intraarterial, parenteral, buccal, transdermal, rectal, intramuscular, subcutaneous, intraosseous, transmucosal, or intraperitoneal routes of administration. In preferred embodiments, topical routes of administering (i.e., "topically applying") a composition are suitable.

The terms "modulate" or "regulate" refer to ability of a pure compound, composition, extract, extract mixture, component of the extract, and/or active agent or ingredient, or a combination thereof of *Dendrobium* to increase, stimulate or enhance, or decrease, prevent or inhibit expression or activity of a specific molecule, thereby reducing or preventing air pollution-induced or air pollution-inducible skin and/or hair damage. For example, the term "modulate" can refer to ability of *Dendrobium* to reduce air pollution-induced oxidative stress, reduce air pollution-induced decrease in cell vitality, reduce air pollution-induced increase in IL-6, and/or reduce air pollution-induced cell damage. The term also refers to Dendrobium's anti-irritant or anti-inflammatory properties to counter potential irritation to skin by air-pollution.

The terms "reduce," "reducing," "inhibit" or "inhibiting" refer to a decrease or reduction in protein activity and/or expression, and/or its downstream effect, in the presence of a plant ingredient or plant extract of *Dendrobium* (e.g., *Dendrobium nobile)* when compared to protein activity and/or expression in the absence of a plant ingredient or plant extract of *Dendrobium* (*Dendrobium nobile*), such as in a control sample. The degree of decrease or inhibition of protein activity and/or expression, and/or its downstream effect, will vary with the nature and quantity of a plant ingredient or plant extract of *Dendrobium* present, but will be evident, e.g., as a detectable decrease in protein activity and/or expression; desirably a degree of decrease greater than 10%, 25%, 50%, 75%, 90%, 95% or 99% as compared to protein activity and/or expression in the absence of *Dendrobium.*

The term "prevent" in the context of preventing air pollution-induced skin and/or hair damage, air pollution-induced oxidative stress, air pollution-induced decrease in cell vitality, air pollution-induced increase in IL-6; and/or air pollution-induced cell damage, and the like in a subject means to keep these effects from happening or existing.

The terms "increase," "increasing," "stimulate," or "stimulating" refer to an increase in in protein activity and/or expression, and/or its downstream effect, in the presence of a plant ingredient or plant extract of *Dendrobium,* when compared to protein activity and/or expression in the absence of a plant ingredient or plant extract of *Dendrobium,* such as in a control sample. The degree of increase of protein activity and/or expression, and/or its downstream effect, will vary with the nature and quantity of a plant ingredient or plant extract of *Dendrobium* present, but will be evident, e.g., as a detectable increase in protein activity and/or expression; desirably a degree of increase greater than 10%, 25%, 50%, 75%, 90%, 95% or 99% as compared to protein activity and/or expression in the absence of a plant ingredient or plant extract of *Dendrobium.*

As used herein, the term "subject" or "individual" includes mammals to which a composition may be administered or applied. Non-limiting examples of mammals include humans, non-human primates, rodents (including transgenic and non-transgenic mice) or the like. In some embodiments, the subject is a mammal, and in some embodiments, the subject is human.

### Compositions

One embodiment relates to a cosmetic composition comprising an isolated ingredient or an extract of *Dendrobium,* e.g., *Dendrobium nobile,* in an amount sufficient for reducing or preventing air pollution-induced or air pollution-inducible skin and/or hair damage (e.g., skin cancer, skin inflammation, and/or hyperpigmentation), reducing or preventing air pollution-induced oxidative stress, reducing or preventing air pollution-induced decrease in cell vitality, reducing or preventing air pollution-induced increase in IL-6, and/or reducing or preventing air pollution-induced cell damage; and, and at least one of pharmaceutically or cosmetically acceptable vehicle.

*Dendrobium* is a large genus of orchids, and today contains about 1,200 species. The genus occurs in diverse habitats throughout much of south, east and southeast Asia, including China, Japan, India, the Philippines, Indonesia, Australia, New Guinea, Vietnam, and many of the islands of the Pacific. The name is from the Greek dendron ("tree") and bios ("life"). *Dendrobium* species are either epiphytic, or occasionally lithophytic. They have adapted to a wide variety of habitats, from the high altitudes in the Himalayan mountains to lowland tropical forests and even to the dry climate of the Australian desert. Exemplary species of *Dendrobium* include *Dendrobium anosmum, Dendrobium aphyllum, Dendrobium begaudii, Dendrobium bilobum, Dendrobium brevicaudum, Dendrobium bukidnonensis, Dendrobium bullenianum, Dendrobium cariniferum, Dendrobium ceraula, Dendrobium chameleon, Dendrobium chrysanthum, Dendrobium conanthum, Dendrobium crispilinguum, Dendrobium crumenatum, Dendrobium cuthbertsonii, Dendrobium dearei, Dendrobium* erosum, *Dendrobium epidendropsis, Dendrobium euryanthum, Dendrobium fairchildae, Dendrobium falcorostrum, Dendrobium forbesii, Dendrobium formosum, Dendrobium gerlandianum, Dendrobium gibsonii, Dendrobium gnomus, Dendrobium goldschmidtianum, Dendrobium guamense, Dendrobium guerreroi, Dendrobium hellwigianum, Dendrobium hercoglossum, Dendrobium heterocarpum, Dendrobium hymenophyllum, Dendrobium ionopus, Dendrobium johnsoniae, Dendrobium junceum, Dendrobium kingianum, Dendrobium lydiae, Dendrobium lindleyi, Dendrobium lineale, Dendrobium lituiflorum, Dendrobium loddigesii, Dendrobium macrophyllum, Dendrobium macropus, Dendrobium milaniae, Dendrobium mindanaense, Dendrobium miyasakii, Dendrobium moniliforme, Dendrobium nobile, Dendrobium papilio, Dendrobium parthenium, Dendrobium pendulum, Dendrobium pentapterum, Dendrobium phalaenopsis, Dendrobium philippinense, Dendrobium phillippsii, Dendrobium platycaulon, Dendrobium polysema, Dendrobium profusum, Dendrobium ramosii, Dendrobium sanderae, Dendrobium sanguinolentum, Dendrobium schuetzei, Dendrobium secundum, Dendrobium seratilabium, Dendrobium sinense, Dendrobium speciosum, Dendrobium taurinum, Dendrobium thyrsiflorum, Dendrobium thysanophorum, Dendrobium uniflorum, Dendrobium velutinalabrum. Dendrobium victoriae-reginae, Dendrobium wangliangii, Dendrobium wenzellii,* and *Dendrobium yeageri.*

In certain embodiments, an isolated ingredient or an extract of *Dendrobium nobile* is used to produce compositions described herein. *Dendrobium nobile* is a very precious and well-known Chinese herb plant with beautiful flowers. Its name comes from its shape; *Dendrobium nobile* looks like an ancient Chinese pin.

The extract of the stems of *Dendrobium nobile* can yield 17 phenanthrenes, including 3,4,8-trimethoxyphenanthrene-2,5-diol, 2,8-dihydroxy-3,4,7-trimethoxyphenanthrene, 3-hydroxy-2,4,7-trimethoxy-9, 10-dihydrophenanthrene, 2,8-dihydroxy-3,4,7-trimethoxy-9, 10-dihydrophenanthrene, 2-hydroxy-4,7-dimethoxy-9, 10-dihydrophenanthrene, 2,2'-dihydroxy-3,3',4,4',7,7'-hexamethoxy-9,9', 10, 10'-tetrahydro- 1,1'-biphenanthrene and 2,3,5-trihydroxy-4,9-dimethoxyphenanthrene (Hwang, Ji Sang, et al., "Phenanthrenes from Dendrobium nobile and their inhibition of the LPS-induced production of nitric oxide in macrophage RAW 264.7 cells," Bioorganic & Medicinal Chemistry Letters, 20 (12):3785 (2010); and Yang, H, et al., "Antifibrotic phenanthrenes of Dendrobium nobile stems," Journal of Natural Products, 70 (12): 1925-9 (2007)).

In certain embodiments, the extract of the stems of *Dendrobium nobile* may be standardized with polysaccharide at about 0.01-80%. In certain alternative embodiments, the extract of the stems of Dendrobium nobile may be standardized with polysaccharide at about 0.1 -0.2% (w/w).

In one example, a botanical extract useful in the unique compositions described herein might be obtained using a deionized water extraction technique.

For example, the polysaccharide extraction may be performed by extracting the stem of *Dendrobium nobile* with deionized water at a temperature (10-100° C), centrifuged and filtered. Next, the extract was concentrated at low temperature in a vacuum. The polysaccharide was purified and concentrated by adding ethanol to obtain deposits-dispersed solvent. Next, the deposits were centrifuged and collected, dissolved in water. A sterilization followed, and the extract was then packaged.

In another example, water extraction and alcohol precipitation methods (0-30% ethanol-aqueous (v/v)) may be used to obtain a botanical extract useful in the unique compositions described herein. Although the botanical extract generated in this process can contain a broad variety of phytochemicals, continually using alcohol-precipitation method results in polysaccharide purification and concentration.

In yet another example, membrane filtration methods may be used to obtain a botanical extract useful in the unique compositions described herein.

The botanical extract generated by the described extraction process(es) will contain a broad variety of phytochemicals present in the extracted material.

In addition, a suitable plant ingredients and botanical extracts of *Dendrobium nobile* may be obtained using any of the extraction and purification techniques discussed more fully below or known in the art.

Those of skill in the art will appreciate that there are many other extraction processes, both known in the art and described in various patents and publications that can be used to obtain the extracts to be used in practicing the present invention.

Application of the topical composition described herein may reduce or prevent air pollution-induced or air pollution-inducible skin and/or hair damage, reduce or prevent air pollution-induced oxidative stress, reduce or prevent air pollution-induced decrease in cell vitality, reduce or prevent air pollution-induced increase in IL-6, and/or reduce or prevent air pollution-induced cell damage.

In certain embodiments, the application of the topical composition described herein may result in reduction of ROS levels by at least about 5%, at least about 10%, at least about 15%, at least about 20%, at least about 21%, at least about 21.5%; at least about 22%, at least about 22.5%; at least about 23%, at least about 23.5%; at least about 24%; at least about 24.5%, at least about 25%, at least about 25.5%, at least about 26%, at least about 26.5%, at least about 27%, at least about 27.5%, at least about 28%, at least about 28.5, at least about 29%, at least about 29.5%, or at least about 30% (percentages in between exemplary percentages provided herein are also contemplated). Higher percentages of reduction are also be contemplated. In certain embodiments, application of the topical composition described herein may result in reduction of ROS levels by about 27.78%.

In certain embodiments, the application of the topical composition described herein may result in reduction of cell damage rate by about 5%, at least about 10%, at least about 15%, at least about 20%, at least about 21 %, at least about 21.5%; at least about 22%, at least about 22.5%; at least about 23%, at least about 23.5%; at least about 24%; at least about 24.5%, at least about 25%, at least about 25.5%, at least about 26%, at least about 26.5%, at least about 27%, at least about 27.5%, at least about 28%, at least about 28.5, at least about 29%, at least about 29.5%, at least about 30%, at least about 30.5%, at least about 31 %, at least about 31.5%, at least about 32%, at least about 32.5%, at least about 33%, at least about 33.5%, at least about 34%, at least about 34.5%, or at least about 35% (percentages in-between the exemplary percentages provided herein are also contemplated). Higher percentages of reduction are also be contemplated. In certain embodiments, application of the topical composition described herein may result in reduction of cell damage rate by about 30.99%.

In certain embodiments, the application of the topical composition described herein may result in reduction of IL-6 levels by about 5%, at least about 10%, at least about 15%, at least about 20%, at least about 21%, at least about 21.5%; at least about 22%, at least about 22.5%; at least about 23%, at least about 23.5%; at least about 24%; at least about 24.5%, at least about 25%, at least about 25.5%, at least about 26%, at least about 26.5%, at least about 27%, at least about 27.5%, at least about 28%, at least about 28.5, at least about 29%, at least about 29.5%, at least about 30%, at least about 30.5%, at least about 31 %, at least about 31.5%, at least about 32%, at least about 32.5%, at least about 33%, at least about 33.5%, at least about 34%, at least about 34.5%, at least about 35%, at least about 35.5%, at least about 36%, at least about 36.5%, at least about 37%, at least about 37.5%, at least about 38%, at least about 38.5%, at least about 39%, at least about 40%, at least about 40.5%, at least about 4 1%, at least about 41.5%, at least about 42%, at least about 42.5%, at least about 43%, at least about 43.5%, at least about 44%, at least about 44.5%, at least about 45% (percentages in-between the exemplary percentages provided herein are also contemplated). Higher percentages of reduction are also be contemplated. In certain embodiments, application of the topical composition described herein may result in reduction of IL-6 levels by about 41.3%.

The compositions described herein may also provide a variety of anti-aging and skin texture benefits as result of the composition's anti-pollution effects. It is believed that a composition comprising *Dendrobium nobile* provides significant anti-pollution effects resulting in significant anti-aging and skin texture benefits relative to other commercially available skin health topical products.

In certain embodiments, the anti-pollution benefits of the composition including *Dendrobium nobile,* manifest in anti-aging and improved aesthetic appearance. Accordingly, certain embodiments relate to topical compositions and methods for their use in treating skin to prevent, arrest, reverse, ameliorate, diminish, reduce or improve signs of aging, including, or associated with, air pollution-induced or air pollution-inducible skin and/or hair damage. The signs of aging may include, but are not limited to, skin fragility; loss of collagen and/or elastin; estrogen imbalance in skin; skin atrophy; appearance and/or depth of lines and/or wrinkles, including fine lines; skin discoloration, including dark eye circles; crow's feet; skin sagging; skin fatigue and/or stress, e.g., skin breakout; skin dryness, fine lines due to skin dryness, skin roughness; skin flakiness; cellular aging; loss of skin tone, elasticity, clarity, luminosity, and/or luster; loss of skin firmness; poor skin texture; loss of skin elasticity and/or resiliency; thin skin, and inflammation.

The benefits and improvements to the aesthetic appearance of skin can be manifested in any of the following: reduction in pore size, fine lines, wrinkles, tactile roughness, and inflammation; improvement in skin tone, radiance, clarity and/or tautness; promotion of anti-oxidant activity; improvement in skin firmness, plumpness, suppleness, and/or softness; improvement in procollagen and/or collagen production; improvement in skin texture and/or promotion of retexturization; improvement in skin barrier repair and/or function; improvement in appearance of skin contours; restoration of skin luster, clarity, and/or brightness; replenishment of essential nutrients and/or constituents in the skin decreased by aging and/or menopause; improvement in communication among skin cells; increase in cell proliferation and/or multiplication; increase in skin cell metabolism decreased by aging and/or menopause; improvement in skin moisturization; promotion and/or acceleration of cell turnover; enhancement of skin thickness; increase in skin elasticity and/or resiliency; and enhancement of exfoliation, with or without the use of alpha or beta hydroxy acids, keto acids or other exfoliants.

Other benefits may include an increase in skin smoothness and/or softness, an increase in the perception of skin condition, an increase in skin moisture, a reduction in skin stress and fine lines, an increase in brightness and/or lightening, improved skin texture and skin firmness.

In one embodiment of the present invention, the topical composition includes an isolated ingredient or an extract of *Dendrobium,* e.g., *Dendrobium nobile* in an amount sufficient to at least reduce and/or prevent air pollution-induced or air pollution-inducible skin and/or hair damage, reduce or prevent air pollution-induced oxidative stress, reduce and/or prevent air pollution-induced decrease in cell vitality, reduce or prevent air pollution-induced increase in IL-6, and/or reduce or prevent air pollution-induced cell damage. *Dendrobium nobile* may be present in an amount ranging from about 0.000 1% to 5% by weight of the total composition; desirably, from about 0.001 % to about 2.5% by weight of the total composition; more desirably, from about 0.001 % to about 0.5% by weight of the total composition; even more desirably, from about 0.01% to about 0.1% by weight of the total composition. The composition may be a personal care composition, a skin care composition, a hair care composition, an anti-aging care composition, or a sun care composition. The compositions may have a pH between about 6.0 to about 8.0, or alternatively the composition may have a pH that is substantially neutral. In certain embodiments, the compositions in which the botanical extract is used have a generally neutral pH.

Certain further embodiments relate to a topical composition that includes *Dendrobium* in the amounts sufficient to provide benefits to skin relating to anti-irritant or anti-inflammatory properties to counter potential irritation to skin by air-pollution.

### Additional Components

In certain other embodiments, the compositions described herein can further include DNA repair enzymes. As used herein, the term "DNA repair enzyme" is intended to include the foregoing enzymes and other enzymes now known or subsequently discovered or developed, including glycosylases, apurinic/apyrimidinic endonucleases or other enzymes having activities capable of repairing damaged DNA. DNA repair enzymes may include enzymes involved in either the base excision repair (BER), the nucleotide excision repair (NER) pathway, or alternate excision repair pathways as described in e.g., U.S. Pat. No. 6,368,594. These pathways are mediated by separate sets of proteins capable of carrying out DNA incision, lesion removal, gap-filling, and ligation reactions.

The NER pathway constitutes a widely distributed, lesion non-specific repair pathway orchestrating DNA damage removal via a dual incision reaction upstream and downstream from the damage site resulting in release of an oligonucleotide containing the damage. Following removal of the damaged DNA, the resulting gap is filled and the DNA ends are ligated together.

The BER pathway is the primary defense against all major forms of DNA base damage. This pathway is responsible for detecting and removing a variety of specific, individual base lesions within a large pool of undamaged DNA. BER pathways typically involve the activity of N-glycosylase/AP lyase enzymes specific for CPDs. The N-glycosylase/AP lyase enzymes first cleave the N-glycosidic bond of a CPD 5' pyrimidine and then cleave the phosphodiester backbone at an abasic site via a β-lyase mechanism.

Suitable DNA repair enzymes for use in combination with an ingredient and/or extract of *Dendrobium* have N-glycosylase/AP lyase activities capable of recognizing, excising and repairing damaged DNA, such as CPDs and (6-4) photoproducts. The activity of these enzymes can be light-dependent (e.g., photolyases) or light-independent. Exemplary DNA repair enzymes in this group include, but are not limited to, bacteriophage T4 pyrimidine dimer-specific endonuclease (denV endonuclease), *Micrococcus luteus* N-glycosylase/AP lyase, *Saccharomyces cerevisiae* N-glycosylase/apurinic-apyrimidinic lyase, *Schizosaccharomyces pombe* UV damage endonuclease (UVDE), *Chlorella* virus isolate PBCV- I pyrimidine dimer-specific glycosylase, *Anacystis nidulans* photolyase, and modified, non-native (e.g., recombinant) enzyme products thereof.

DNA repair enzymes may also include other members from the BER, NER or alternate pathways. These enzymes may include O⁶-methylguanine-DNA methyltransferases, uracil- and hypoxanthine-DNA glycosylases, DNA exonucleases, damaged-bases glycosylases (e.g., 3-methyladenine-DNA glycosylase), endonucleases alone or in complexes (e.g., *E*. *coli* uvrA/uvrB/uvrC endonuclease complex), and other enzymes and enzyme complexes whose activities at present are only partially understood, such as, the products of the ERCC genes of humans and the RAD genes of yeast. Exemplary DNA repair enzymes include, but are not limited to, uracil DNA glycosylases, 3-methyladenine DNA glycosylase, Endonuclease III/thymine glycol DNA glycosylases, Endonuclease VIII, fapy/8-oxoguanine DNA glycosylases, A-G-mismatch DNA glycosylases, G-T mismatch DNA glycosylases, formyluracil DNA glycosylases, hydroxymethyl uracil DNA glycosylases, XPC-hHR23B, XPA, RPA, XPB, TFIIH, XPG, XPF-ERCC I, Rad-4-Rad23, Rad14, Rfa, Rad25/Ss12, Rad3, Rad2, Rad1-Rad10, various DNA polymerases, DNA ligases and the like. Exemplary sources for these enzymes may include bacterial or mammalian cell sources, including, but not limited to *E. coli, S. cerevisiae, S. pombe,* human, human, monkey, mouse, rat, hamster and the like.

DNA repair enzymes may be derived or extracted from suitable sources such as *E. coli, Micrococcus,* and the like. The DNA repair enzymes may be encapsulated in liposomes as described in U.S. Pat. No. 5,296,231, the entire content of which is incorporated herein by reference. For example, a DNA repair enzyme derived from a *Micrococcus luteus* cell lysate is provided in a liposomal formulation containing lecithin and water and is available as ULTRASOMES™ from Applied Genetics, Inc. Dermatics, Freeport, N.Y. or ULTRASOMES-V™ from Barnet Products Corporation, Englewood Cliffs, N.J. Liposomes encapsulating an *Anacystis nidulans* lysate containing the *Anacystis nidulans* photolyase are available as PHOTOSOMES™ or PHOTOSOMES-V™ from Applied Genetics, Inc. Dermatics, (Freeport, N.Y.). The liposomes may include conventional phospholipids, oleic acid and/or cholesterol hemisuccinate from vegetable-derived sources, e.g., soybean or they may be produced from other suitable sources conventionally known to those skilled in the art.

Exemplary embodiments may incorporate ULTRASOMES™, ULTRASOMES-V™, PHOTOSOMES™, or PHOTOSOMES-V™ in an amount ranging from about 0.01% to 20% by weight of the total composition, desirably from about 0.1wt % to about wt 10%, more desirably from about 0.5 wt % to about 3 wt %.

Liposomes may be used as delivery agents to facilitate transfer of cosmetically active agents into the dermis of skin, such as the plant or botanical ingredients of the present invention and the DNA repair enzymes. Other delivery agents may be used for dermal delivery in place of the liposomes, including, but not limited to skin delivery vehicles known to those skilled in the art, including emulsions, microemulsions, nanoemulsions, nanoparticles, microspheres, ethosomes, transfersomes, and niosomes.

In certain embodiments, additional cosmetic ingredients may also be included in the cosmetic composition described herein, including, but not limited to, ingredients present in: licorice, licorice extracts, licorice derivatives (e.g., glycyrrhizinates); lemon extract; cucumber extract; sunflower seed extract; castor seed oil; oat proteins, oat extracts, hydrolyzed oats; silk protein (e.g., sericin); hyaluronic acid and its derivatives (e.g., sodium hyaluronate); vitamins; minerals; anti-oxidants; phospholipids, sphingolipids, cholesterol; and/or other ingredients or combinations thereof having anti-aging, anti-oxidant, anti-inflammatory, anti-irritant, anti-cancer or other skin-protective properties; aesthetic appearance enhancing properties; and/or increased skin delivery properties.

Cosmetically useful vitamins, minerals and/or anti-oxidants for topical application in accordance with the present invention include plant ingredients and extracts having anti-oxidant properties (e.g., Rosemary extract, *Centella asiaticoside,* etc.); vitamin A and its precursors or derivatives (e.g., beta-carotene, retinyl palmitate); vitamin B3 and its precursors or derivatives (e.g., niacinamide); vitamin B5 and its precursors or derivatives (e.g. panthenol); vitamin C and its precursors or derivatives (e.g., tetrahexyldecyl ascorbate, ascorbyl palmitate); vitamin E and its precursors or derivatives (e.g., d-alpha-tocopherol, tocopheryl acetate); vitamin K and its precursors or derivatives; selenium and its derivatives (e.g., L-selenomethionine); and alpha lipoic acid (ALA).

ALA is a potent, naturally occurring anti-oxidant, sometimes referred to as the "universal anti-oxidant" because of its activity and solubility in both water and lipids. ALA is able to penetrate into skin cells, is able to prevent activation of the proinflammatory NF-kB pathway responsible for breakdown of collagen and elastin, and is able to boost the protective effects of vitamins E and C, thereby boosting naturally occurring anti-oxidants within cells.

In one embodiment, tetrahexyldecyl ascorbate may be incorporated in the composition described herein. Tetrahexyldecyl ascorbate is a stable, lipid-soluble ester derivative of vitamin C. Vitamin C has been reported to promote collagen synthesis, inhibit lipid breakdown, regenerate vitamin E, reduce fine lines and wrinkles, heal sunburns, and is a potent anti-oxidant scavenger of free radicals having significant anti-inflammatory properties, hindering production of e.g., arachidonic acid.

In another embodiment, panthenol or its equivalents are contemplated for use with the composition. Panthenol is an effective film-forming moisturizing agent having anti-inflammatory properties. Panthenol equivalents may include alcohol derivatives of pantothenic acid, such as the ones described in CTFA Cosmetic Ingredient Handbook, The Cosmetic, Toiletry and Fragrance Association. Inc., pp. 272-273, 1992. For optimal usefulness, the amount of panthenol should be chosen so that the composition dries reasonably quickly. The more panthenol in the composition, the longer it takes for the composition to dry when it is applied to skin or other surfaces.

Vitamins, minerals, and/or anti-oxidants may be present in a collective amount ranging from about 0.01 % to 20% by weight of the total composition, desirably from about 0.1wt % to about wt 10%, more desirably from about 0.5 wt % to about 3 wt %.

Optionally, the present composition may additionally include one or more anesthetics, anti-allergenics, anti-irritants, antifungals, anti-microbials, anti-inflammatory agents, antiseptics, chelating agents, colorants, depigmenting agents, emollients, emulsifiers, film formers, fragrances, humectants, insect repellents, lubricants, moisturizers, pharmaceutical agents, photostabilizing agents, preservatives, skin protectants, skin penetration enhancers, sunscreens, stabilizers, surfactants, thickeners, viscosity modifiers, or any combinations thereof.

Although some embodiments lack the use of exfoliating agents, these agents may be included provided that sufficient anti-irritant/anti-inflammatory agents are included to ameliorate the irritating effects of exfoliating agents. Exemplary anti-irritants include, but are not limited to, aloe vera, α-bisabolol, caffeine or other xanthenes, chamomile, cola nitada extract, dipotassium glycyrrhizinate, glycyrrhizic acid and its derivatives, green tea extract, lecithin or hydrogenated lecithin, licorice extract, tea tree oil, steroidal or non-steroidal anti-inflammatory agents, including, but not limited to cyclooxygenase inhibitors (e.g., salicylic acid, acetylsalicylic acid), NF-κB inhibitors, strontium acetate, strontium chloride, strontium nitrate, urea, or combinations thereof. Desirable anti-irritants may include dipotassium glycyrrhizinate, lecithin and hydrogenated lecithin.

Anti-irritant or anti-inflammatory agents may be present individually or collectively in an amount ranging from about 0.01 % to 10% by weight of the total composition, desirably from about 0.05 wt % to about 5 wt %, more desirably from about 0.2 wt % to about 1.5 wt %.

The plant ingredients, plant extracts, oils, vitamins, minerals, antioxidants, anti-irritants or other active agents may be included, either individually or collectively, in a pharmaceutically or cosmetically acceptable vehicle. Examples of pharmaceutically or cosmetically acceptable vehicles suitable for the embodiments of the present invention include, but are not limited to, water, C1-C4 alcohols, fatty alcohols, fatty ethers, fatty esters, glycerin, glycols, vegetable oils, mineral oils, lecithin, hydrogenated lecithin, liposomes, laminar lipid materials, phospholipids, polyglycols, polyols, propyl alcohol, silicone oils, vegetable oil, or any combinations thereof.

The pharmaceutically or cosmetically acceptable vehicle for use with the compositions described herein may be in the form of a homogeneous phase formulation or in the form of an emulsion or microemulsion including, but not limited to, oil-in-water, water-in-oil and multiple including triple, phase emulsions. These emulsions can cover a broad range of consistencies including thin lotions (which can also be suitable for spray or aerosol delivery), creamy lotions, light creams and heavy creams. Other suitable topical carriers include anhydrous liquid solvents such as oil and alcohol; aqueous-based single phase liquid solvent (e.g., hydro-alcoholic solvent system); anhydrous solid and semisolid (such as gel and stick); and aqueous based gel and mousse system.

The pharmaceutically or cosmetically acceptable vehicle will usually contain from about 5% to about 99.9% by weight of the total composition, desirably from about 25% to about 80%, and more desirably from about 50% to about 70% by weight of the composition, and may, in the absence of other cosmetic adjuncts, form the balance of the composition. Any percentage in between 5% and 99% by weight of the total composition is also contemplated.

Emollients are moisturizers to maintain hydration or to rehydrate the skin by providing a protective emollient coating. Emollients may be classified under such general chemical categories as esters, fatty acids and alcohols, polyols and hydrocarbons. Esters may be mono- or di-esters. Representative examples of fatty di-esters include, but are not limited to, dipotassium glycyrrhizinate, dibutyl adipate, diethyl sebacate, diisopropyl dimerate, and dioctyl succinate. Acceptable branched chain fatty esters include, but are not limited to, 2-ethyl-hexyl myristate, isopropyl stearate and isostearyl palmitate. Acceptable tribasic acid esters include, but are not limited to, triisopropyl trilinoleate and trilauryl citrate. Acceptable straight chain fatty esters include, but are not limited to, lauryl palmitate, myristyl lactate, and stearyl oleate.

Suitable fatty alcohols and acids may include, but are not limited to, alcohols or acids having from about 10 to 20 carbon atoms. For example, alcohols such as cetyl, myristyl, palmitic and stearyl alcohols and acids may be used.

Polyols may serve as emollients, including, but not limited to linear and branched chain alkyl polyhydroxyl compounds. Representative polyols, include, but are not limited to butylene, propylene glycol, sorbitol, glycerin, polymeric polyols, such as polypropylene glycol and polyethylene glycol, and the like.

Hydrocarbons may serve as emollients and may include hydrocarbon chains having from about 12 to 30 carbon atoms, including, but not limited to mineral oil, petroleum jelly, squalene and isoparaffins.

Exemplary emollients include, but are not limited to, butylene, caprylic/capric triglyerides, castor oil, ceteareth-20, ceteareth-30, cetearyl alcohol, ceteth 20, cetostearyl alcohol, cetyl alcohol, cetyl stearyl alcohol, cholesterol, cocoa butter, diisopropyl adipate, glycerin, gyceryl monooleate, glyceryl monostearate, glyceryl stearate, isoparaffins, isopropyl myristate, isopropyl palmitate, lanolin, lanolin alcohol, hydrogenated lanolin, isoparaffins, liquid paraffins, linoleic acid, mineral oil, oleic acid, petroleum jelly, phospholipids, polyethylene glycol, polyethylene glycol-7 glyceryl cocoate, polyethylene glycol- 18 methyl ester dimethyl silane, polyoxyethylene glycol fatty alcohol ethers, polyoxypropylene 15-stearyl ether, polypropylene glycol propylene glycol, propylene glycol stearate, sorbitol, sphingolipids, squalene, steareth-2 or -100, stearic acid, stearyl alcohol, urea, white petrolatum, and the like.

Emollients may be present individually or collectively in an amount ranging from about 0.005% to 20% by weight of the total composition, desirably from about 0.1 wt % to about 10 wt %, more desirably from about 1.0 wt % to about 5.0 wt %.

Humectants are moisturizers that can bind water and retain it on the skin surface. Exemplary humectants include, but are not limited to, acetyl glucosamine, bisaccharide gum, butylene glycol, ethoxydiglycol, ethylene glycolpolypropylene, glucose, glycereth-26, glycerin, glycerol, glycol, lactitol, maltitol, propylene glycol, sericin, sodium hyaluronate, sorbitol, xylitol, sodium citrate, glucose and the like.

Humectants may be collectively present in an amount ranging from about 0.1% to 40% by weight of the total composition, desirably from about 2.5 wt % to about wt 25%, more desirably from about 5 wt % to about 15 wt %.

The present compositions may include one or more preservatives. Suitable preservatives include disodium EDTA, benzyl alcohol, methylparaben, phenoxyethanol, propylparaben, ethylparaben, butylparaben and isobutylparaben.

Another category of functional ingredients within the compositions are thickeners. Thickeners will usually be present in a collective amount ranging anywhere from about 0.01 to 10% by weight, desirably from about 0.05 to 5% by weight, more desirably from about 0.1% to I % by weight of the composition. Exemplary thickeners are crosslinked polyacrylate materials. Gums such as xanthan, carrageenan, gelatin, karaya, pectin and locust bean gum may be used. Under certain circumstances the thickening function may be accomplished by a material also serving as a silicone or emollient. For instance, silicone gums in excess of 10 centistokes and esters such as glycerol stearate have dual functionality.

In certain embodiments skin whitening agents may be included in the described compositions. Skin whitening agents include but are not limited to tyrosinase inhibitors, free radical scavengers, and mixtures thereof.

Powders may be incorporated into the cosmetic composition. These powders include chalk, talc, kaolin, starch, smectite clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into the described cosmetic compositions. These ingredients may include coloring agents, opacifiers and perfumes. Amounts of these other adjunct minor components may range anywhere from 0.001 % up to 20% by weight of the composition.

The compositions of the present invention may be formulated in any convenient form suitable for topical application to the skin. Such product forms include, but are not limited to, aerosol spray, cream, dispersion, emulsion, foam, gel, liquid, lotion, mousse, ointment, patch, pomade, pack or powder, pump spray, solid, solution, stick, and towelette.

A desired cosmetic form is a cream that is an oil-in-water emulsion. Water-in-oil and water-in-silicone emulsions also are contemplated. In each formulation, various known conventional cosmetic ingredients may be incorporated. For example, cosmetic ingredients such as alcohols, fats and oils, surfactants, fatty acids, silicone oils, humectants, moisturizers, viscosity modifiers, emulsifiers, stabilizers, coloring agents, and perfumes may be included.

The compositions may be administered as needed, daily, several times per day or in any suitable regimen, such that the desired outcome is achieved.

The compositions may be administered on a continuous basis or intermittent basis.

Certain embodiments relate to a cosmetic treatment system including a patch impregnated with the topical composition of the present invention. Patches for use in the present invention may come in any shape suitable for treating a particular target area. The patch may encompass a small area targeting a particular area or it may cover a large area, such as a face in the form of a mask. The overall size and geometry of current patches for applying medicaments around the eyes can make it difficult to apply eye treatment products in close proximity to the eye. A patch may be made of any removal material suitable for absorbing, containing, and releasing compositions of the present invention. For example, the patch may be made of non-woven material. The non-woven material may include cotton, cotton/polyester blends, or other suitable combinations of natural or synthetic materials. The patch may be further adapted to provide an occlusive, semi-occlusive or non-occlusive barrier. The patch may be adhesive or non-adhesive. The patch may include a single layer of material or it may include multiple layers of the same and/or dissimilar materials to provide additional structural integrity and/or flexibility. Suitable patches or patch materials are disclosed in, e.g., U.S. Pat. Nos. 6,096,334; 6,120,792; 6,495,158; 6,623,751; 8,697,099; U.S. Pat. Appl. No. 2002/0086043; U.S. Pat. Appl. No. 2003/01526 10; U.S. 2003/0175328; and references cited therein, the contents of which are incorporated herein by reference.

In certain embodiments, the cosmetic treatment system of the present invention may include a packaging system for holding the individual components of the cosmetic treatment system. In a preferred embodiment, the cosmetic treatment system includes a patch; at least one container; and a topical composition formulated for treatment of at least a portion of the face.

One or more containers may be used to hold one or more of the components of the cosmetic treatment system. Any container(s) suitable for holding the components of the cosmetic treatment system may be used in accordance with conventional practices known to those skilled in the art. Alternatively, the patch may be packaged in a container in the form of a sunken tray overlayed with a sealably removable cover to securely maintain the patch in the sunken tray prior to use.

The container may be prepared by thermoforming or by thin-wall injection molding of a suitable material, such as polypropylene. The design of the container can be modified and adapted to the shape of the particular patch. Suitable containers are disclosed in U.S. Pat. No. 6,623,751, the contents of which are incorporated herein by reference.

Certain further embodiments relate to a cosmetic treatment system including a packaging system containing a suitable amount of the cosmetic composition suitable for a desired period of time, such as fourteen days. According to this embodiment, the cosmetic treatment system includes a packaging system containing a plurality of containers, with each container having an amount of the cosmetic composition according to the present invention suitable for a single use. The container may be in the form of a vial or other suitable holding device.

In one embodiment, the cosmetic treatment system includes a packaging system having a plurality of vials, each vial containing a sufficient amount of the cosmetic composition suitable for a single application of the cosmetic composition to the skin. The packaging system may be formulated to provide a number of vials matching the number of days in which the cosmetic composition is applied to skin. Alternatively, the packaging system may be formulated for more than one application per day. In one embodiment, the packaging system may contain 14 vials for daily treatment to skin over a period of 14 days. The packaging system may further contain one or more applicators for applying the compositions and may further include a set of instructions for use of the packaging system associated with the cosmetic treatment system.

The present invention also includes methods of treating skin by topically applying the cosmetic compositions of the present invention. In use, a small quantity of the composition, for example from 0.1 to 100 mL, may be applied to exposed areas of the skin, from a suitable container or applicator and, if necessary, it is then spread over and/or rubbed into the skin using the hand or fingers or a suitable device. Alternatively, the composition may be applied to the skin in the form of a patch that has been impregnated with the composition. The patch may be made of non-woven material and may further contain an adhesive to adhere the patch to the skin.

Certain additional embodiments relate to a medicament comprising an isolated ingredient or an extract of *Dendrobium,* e.g., *Dendrobium nobile* for preventing, treating and/or curing human skin and/or hair from at least one of: air pollution-induced or air pollution-inducible skin and/or hair damage, air pollution-induced oxidative stress, air pollution-induced decrease in cell vitality, air pollution-induced increase in IL-6, and/or air pollution-induced cell damage. An ingredient or an extract of *Dendrobium nobile* may be present in an amount ranging from about 0.000 1% to 5% by weight of the total medicament; desirably, from about 0.001 % to about 2.5% by weight of the total medicament; more desirably, from about 0.001 % to about 0.5% by weight of the total medicament; even more desirably, from about 0.01 % to about 0.1% by weight of the total medicament. The medicaments described herein may also include any additional components listed above in connection with the topical compositions described herein.

Application of medicament described herein may reduce or prevent air pollution-induced or air pollution-inducible skin and/or hair damage, reduce or prevent air pollution-induced oxidative stress, reduce or prevent air pollution-induced decrease in cell vitality, reduce or prevent air pollution-induced increase in IL-6, and/or reduce or prevent air pollution-induced cell damage. The medicaments described herein may also provide a variety of anti-aging and skin texture benefits as result of the composition's anti-pollution effects. It is believed that a composition comprising *Dendrobium nobile* provides significant anti-pollution effects resulting in significant anti-aging and skin texture benefits relative to other commercially available skin health topical products. Exemplary benefits of the medicament described herein include those described above in connection with the topical compositions described above.

### Methods

Certain further embodiments relate to a method for protecting human skin and/or hair against air pollution-induced damage comprising applying a composition or a medicament comprising an isolated ingredient or an extract of *Dendrobium,* e.g., *Dendrobium* nobile, wherein the composition or the medicament is in a working amount sufficient for at least one of: reducing and/or preventing air pollution-induced or air pollution-inducible skin and/or hair damage, reducing and/or preventing air pollution-induced oxidative stress, reducing and/or preventing air pollution-induced decrease in cell vitality, reducing and/or preventing air pollution-induced increase in IL-6, and/or reducing or preventing air pollution-induced cell damage. An ingredient or an extract of *Dendrobium nobile* may be present in an amount ranging from about 0.000 1% to 5% by weight of the total medicament; desirably, from about 0.001 % to about 2.5% by weight of the total medicament; more desirably, from about 0.001 % to about 0.5% by weight of the total medicament; even more desirably, from about 0.01 % to about 0.1% by weight of the total medicament.

Certain further embodiments relate to the use of an isolated ingredient or an extract of *Dendrobium,* e.g., *Dendrobium nobile* as an anti-pollution agent, respectively anti-pollution mixture for protecting human skin and/or hair. The anti-pollution is especially directed to protection and/or inhibition and/or reduction of disorders and dysfunctions of human skin and hair related to, respectively associated with the induction of oxidative stress, decrease in cell vitality, and/or an increased IL-6 gene expression.

Certain other embodiments relate to a method for improving the aesthetic appearance of skin and/hair damaged by air pollution, in which a composition or a medicament described herein is topically applied to skin in a cosmetically or pharmaceutically effective amount, respectively, sufficient to improve the aesthetic appearance of the skin. The improvements may relate to skin aging, lines, fine lines, wrinkles, crow's feet, dark eye circles, blemishes, age spots, dark spots, stretch marks, cancer (including skin cancer), skin inflammation, skin discoloration, skin hyperpigmentation, skin thickness, elasticity, resiliency, moisturization, tone, texture, radiance, luster, lightening, brightness, clarity, contour, firmness, tautness, suppleness, softness, sensitivity, pore size, skin barrier function, or combinations thereof.

The improvements may further relate to improving adverse skin conditions affected by, resulting in or resulting from air pollution that include psoriasis, eczema, seborrhea, dermatitis, sunburn, estrogen imbalance, hyperpigmentation, hypopigmentation, discoloration, yellowing, freckles, skin atrophy, skin breakout, skin fragility, dryness, chapping, sagginess, thinning, hyperplasia, fibrosis, enlarged pores, cellulite formation, bruising, acne formation, apoptosis, cellular differentiation, cellular de-differentiation, prevention of tumor induction or tumor progression, viral infections, fungal infections, bacterial infections, spider veins (telangectasia), hirsutism, rosacea, pruritis, calluses, warts, corns, skin barrier function damage, or combinations thereof.

In addition to having anti-pollution effect on skin and/or hair, the topical compositions or medicaments described herein may be topically applied to enhance the general health, vitality and appearance of the skin. For example, the present composition may be applied to skin to improve microcirculation, communication among skin cells, replenishment of essential nutrients or skin constituents, or to improve the metabolism, proliferation, multiplication, turnover and/or exfoliation of skin cells.

Certain other embodiments relate to a cosmetic treatment system comprising a topical composition including a plant ingredient, plant extract or a natural complex of *Dendrobium,* e.g., *Dendrobium nobile* or an extract thereof, and a pharmaceutically or cosmetically acceptable vehicle.

The following are non-limiting examples of the present invention. Unless indicated otherwise, all proportions and percentages are by weight.

### EXAMPLES

The following are examples of formulations and methods according to the present invention.

### Example I: Preparation of the Dendrobium nobile Extract

A botanical extract of *Dendrobium nobile* was obtained using a deionized water extraction technique.

Specifically, the polysaccharide extraction was performed by extracting the stem of *Dendrobium nobile* with deionized water at a low temperature (about 80°C), followed by centrifugation and filtration using a 10µm membrane. Next, the polysaccharide extract was concentrated also at a low temperature (about 60°C) in a vacuum. The polysaccharide extract was purified and concentrated by adding some ethanol to the point of about 80% (v/v) to obtain deposits-dispersed solvent. Next, the deposits were centrifuged and collected, dissolved in water. A sterilization followed, and the extract was then packaged.

The extract of the stems of *Dendrobium nobile* was standardized with polysaccharide at about 0.1 -0.2% (w/w).

### Example 2: Cytotoxicity of Dendrobium nobile Extract

Cell Counting Kit-8 (CCK-8) provides a tool for studying induction and inhibition of cell proliferation in any in vitro model. CCK-8 allows very convenient assays by utilizing highly water-soluble tetrazolium salt. WST-8 [2-(2-methoxy-4-nitrophenyl)-3-(4-nitrophenyl)-5-(2,4- disulfophenyl)-2H-tetrazolium, monosodium salt] produces a water-soluble formazan dye upon reduction in the presence of an electron mediator. The amount of formazan dye is directly proportional to the number of living cells.

CCK-8 colorimetric assay was performed according to the manufacturer's instructions using Cell Counting Kit-8 purchased from Dojindo Laboratories (Cell Counting Kit-8, Dojindo Laboratories, Kumamoto, Japan) to determine cell viability in cell cytotoxicity assay following a treatment with *Dendrobium nobile* extract as compared to the treatment with PM2.5 particles. PM2.5 particles are air pollutants with a diameter of about 2.5 micrometers or less collected from air.

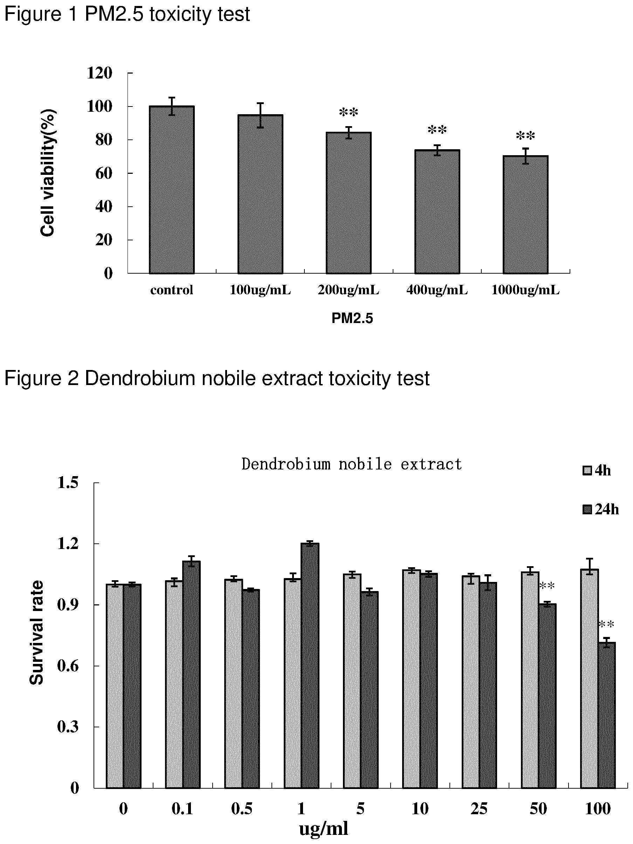

A concentration causing the cell viability 10% lower than control is defined as a cytotoxic standard. The concentration for the PM2.5 particles group was selected so that it will result in cell viability of PM2.5 treatment group of above 80% of controls at 4 hours, as shown in Figure 1. The concentration for the *Dendrobium nobile* extract treatment group was selected so that it will result in cell survival rate of dendrobium nobile extract treatment group of above 80% of controls at 24 hours, while there is no toxic effect at 4 hours, as shown in Figure 2.

**Table 1: Dosage for further testing; µg/mL.**

| ***Denrobium nobile* extract** | **PM2.5** |
|---|---|
| 50 | 200 |

According CCK-8 assay screening, the concentration for the *Dendrobium nobile* extract treatment group and PM2.5 particles treatment group are selected for the following test, as shown in Table 1.

Several in vitro efficacy tests for *Dendrobium nobile* extract as compared to anti-PM2.5 were carried out based on concentrations selected for the cytotoxicity testing (Example 2).

### Example 3: In Vitro Efficacy Testing

To determine the effect of *Dendrobium nobile* extract on the reactive oxygen species (ROS) level in Hacat cells, the Fluorometric Intracellular ROS Kit purchased from Sigma-Aldrich was used (Fluorometric Intracellular ROS Kit, Orange Fluorescence, Catalog Number MAK144).

ROS are generates as a result of the reduction of oxygen during aerobic respiration and by various enzymatic systems within the cell. At physiological levels, ROS contribute to cell signaling and host defense. Increased ROS generation, above the detoxification capacity of the biological system, results in oxidative stress and cellular damage. The main damage to cells results from the ROS-induced alteration of macromolecules such as polyunsaturated fatty acids in membrane lipids, essential proteins, and DNA.

The kit, as noted by the manufacturer, provides a sensitive assay to detect intracellular ROS (especially superoxide and hydroxyl radicals) in live cells within I hour incubation. ROS react with a cell-permeable sensor, resulting in a fluorometric product proportional to the amount of ROS present.

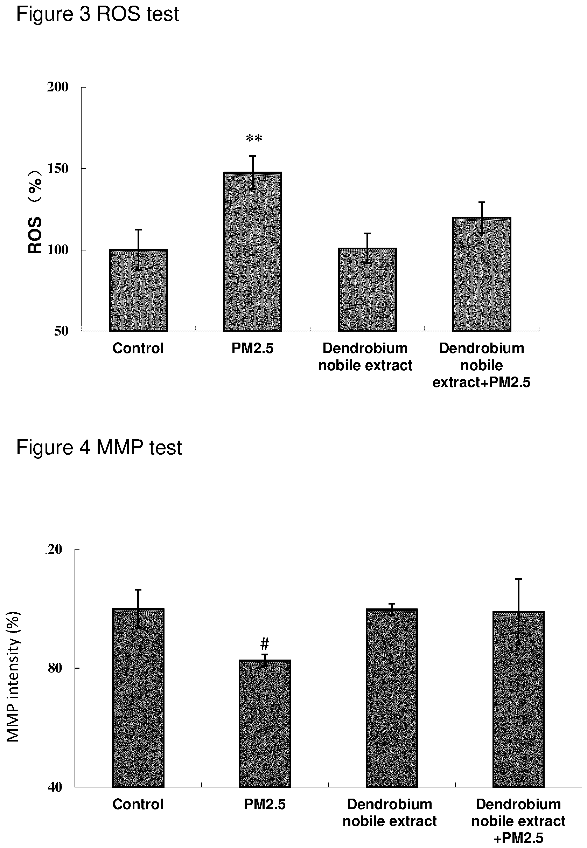

Figure 3 shows that PM2.5 can significantly enhance the ROS level in Hacat cells. In other words, PM2.5 can improve the oxidative stress of Hacat cell. Figure 3 also demonstrates that *Dendrobium nobile* extract can effectively counter the ROS levels increased by PM2.5, and the *Dendrobium nobile* extract itself will not cause oxidative stress of Hacat cell.

### Example 4: Mitochondrial Membrane Potential (MMP) Testing

Figure 4 shows mitochondria membrane potential (MMP) test results. MMP testing was conducted following the manufacturer's instructions using Mitochondria Membrane Potential Kit purchased from Sigma-Aldrich (Mitochondria Membrane Potential Kit, JC-10 Assay for Microplate Readers, Catalog Number MAK159, Sigma-Aldrich, St. Louis, MO). The kit utilizes JC-10 for determining the loss of the MMP in cells. The kit can be used for monitoring apoptosis and for screening apoptosis inhibitors and activators.

As shown in Figure 4, PM2.5 significantly decreased the MMP level and caused a decrease in cell vitality. While *Dendrobium nobile* extract itself does not decrease cell MMP levels, as shown in the figure, *Dendrobium nobile* extract clearly protects cell vitality.

### Example 5: IL-6

This study was designed to determine the effect of *Dendrobium nobile* extract on IL-6 levels as compared to control samples. An ELISA Kit purchased from Sigma-Aldrich was used according to the manufacturer's instructions.

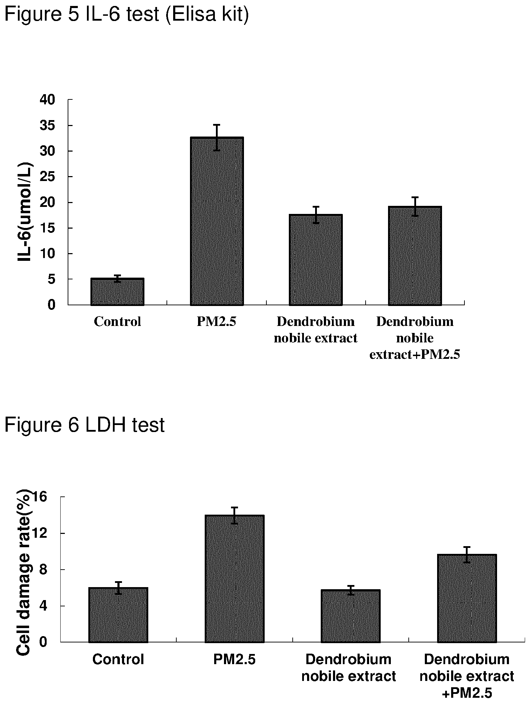

Figure 5 shows IL-6 levels can be induced by various samples (control, PM2.5, *Dendrobium nobile* extract (50ug/ml), and *Dendrobium nobile* extract (50ug/ml )+ PM2.5). When comparing *Dendrobium nobile* extract samples with PM2.5 control samples, PM2.5 significantly increased IL-6 levels. Although *Dendrobium nobile* extract can also increase IL-6 levels at some concentrations, it was shown that *Dendrobium nobile* extract significantly decreases IL-6 levels, by 41.3%, as compared with the PM2.5 treatment group.

### Example 6: Lactate dehydrogenase (LDH or LD)

Lactate dehydrogenase (LDH or LD) is an enzyme found in nearly all living cells (animals, plants, and prokaryotes). LDH catalyzes the conversion of lactate to pyruvic acid and back, as it converts NAD+ to NADH and back. LDH is expressed extensively in various body tissues, such as blood cells and heart muscle. Because it is released during cell damage, it functions as a marker of cell damage. Specifically, the higher the LDH level in the tissue, the more damage cell damage is observed.

A Lactate Dehydrogenase Activity Assay kit purchased from Sigma-Aldrich (Sigma Aldrich, Catalog Number MAK0660) to study cell damage following treatment with PM2.5 and *Dendrobium nobile* extract.

As shown in Figure 6, PM2.5 increases cell damage rate, while treatment with *Dendrobium nobile* extract does not damage cells. Rather, treatment with *Dendrobium nobile* extract can surprisingly prevent cells from damage caused by PM2.5, as shown in the dark green column.

### Example 7: Formulation

An exemplary cosmetic formulation may be as follows:

| Phase | Raw Material | Percentage |
|---|---|---|
| A | Deionized Water | To 100 |
| | Disodium EDTA | 0.05 |
| | Dipotassium Glycyrrhizate | 0.05 |
| | Acrylates/C 10-30 Alkyl Acrylate Cross polymer | 0.1 |
| | 1,3-Butanediol | 3 |
| | Glycereth-26 | 2 |
| B | Deionized Water | 0.075 |
| | Triethanolamine | 0.075 |
| C | Glycerin | I |
| | Xanthan Gum | 0.2 |
| | Bis-PEG-18 Methyl Ether Dimethyl Silane | 0.7 |
| | PEG-40 Hydrogenated Castor Oil | 0.5 |
| | Dendrobium Nobile extract | 0.2 |
| D | Propylene Glycol | 0.45 |
| | Diazolidinyl Urea (and) Iodopropynyl Butylcarbamate | 0.15 |

Based on the Examples 1-5, clearly, *Dendrobium nobile* extract has very good anti-pollution properties. In addition, it is water soluble, and can be used widely in cosmetic products.

It is therefore intended that the foregoing detailed description be regarded as illustrative rather than limiting, and that it be understood that it is the following claims, including all equivalents, that are intended to define the spirit and scope of this invention.

## Claims

1. A cosmetic composition comprising:
an isolated ingredient or an extract of *Dendrobium nobile,* in an amount sufficient for at least one of:
(a) reducing or preventing air pollution-induced or air pollution-inducible skin and/or hair damage;
(b) reducing or preventing air pollution-induced oxidative stress;
(c) reducing or preventing air pollution-induced decrease in cell vitality;
(d) reducing or preventing air pollution-induced increase in IL-6; or
(e) reducing or preventing air pollution-induced cell damage; and
a cosmetically acceptable carrier or excipient.

2. The composition of claim 1, wherein the skin damage is skin aging, lines, fine lines, wrinkles, crow's feet, dark eye circles, blemishes, age spots, dark spots, stretch marks, skin cancer, skin inflammation, skin discoloration, skin hyperpigmentation, skin barrier function damage, or combinations thereof.

3. The composition of any of claims I to 2, wherein the composition is a personal care composition, a skin care composition, a hair care composition, an anti-aging care composition, a sun care composition, or a combination thereof.

4. The composition of any of claims I to 3, wherein the composition is in a form of an aerosol, a cream, a emulsion, a solid, a liquid, a dispersion, a foam, a gel, a lotion, a mousse, an ointment, a powder, a patch, a pomade, a solution, a pump a spray, a stick, am oil, a towelette, or combinations thereof.

5. The composition of any of claims I to 4, wherein the ingredient or the extract of *Dendrobium nobile* is present in an amount of from about 0.000 1% to about 5% by weight of the total composition.

6. The composition of any of claims I to 4, wherein the ingredient or the extract of *Dendrobium nobile* is present in an amount of from about 0.01 % to about 0.1% by weight of the total composition.

7. The composition of any of claims I to 6, wherein the cosmetically acceptable carrier or excipient is selected from the group consisting of water, a glycerin, a C1-C4 alcohol, a fatty alcohol, a fatty ether, a fatty ester, a polyol, a glycol, a vegetable oil, a mineral oil, a liposome, a laminar lipid material, a silicone oil, or combinations thereof.

8. A medicament comprising an ingredient or an extract of *Dendrobium nobile* and a pharmaceutically acceptable carrier or excipient, wherein the medicament is for preventing, treating and/or curing human skin and/or hair from at least one of:
(a) air pollution-induced or air pollution-inducible skin and/or hair damage;
(b) air pollution-induced oxidative stress;
(c) air pollution-induced decrease in cell vitality;
(d) air pollution-induced increase in IL-6; or
(e) air pollution-induced cell damage.

9. The medicament of claim 8, wherein the skin damage is skin aging, lines, fine lines, wrinkles, crow's feet, dark eye circles, blemishes, age spots, dark spots, stretch marks, skin cancer, skin inflammation, skin discoloration, skin hyperpigmentation, skin barrier function damage, or combinations thereof.

10. The medicament of any of claims 8 to 9, wherein the ingredient or the extract of *Dendrobium nobile* is present in an amount of from about 0.000 1% to about 5% by weight of the total composition.

11. The medicament of any of claims 8 to 9, wherein the ingredient or the extract of *Dendrobium nobile* is present in an amount of from about 0.01 % to about 0.1% by weight of the total composition.

12. A packaging system comprising at least one of:
(a) one or more containers collectively containing the composition of any of claims 1-7 or the medicament of any of claims 8-11; or
(b) instructions for applying the composition from the one or more containers.

13. A method for protecting human skin and/or hair against air pollution-induced damage comprising topically applying the composition of any of claims I to 7 or the medicament of any of claims 8-11 to the skin and/or hair.

14. The method of claim 13, wherein an ingredient or an extract of *Dendrobium nobile* is applied to human skin and/or hair in an effective amount of about 0.01 to about 5 mg/cm².

15. The use of an ingredient or an extract of *Dendrobium nobile* as an anti-pollution agent for protecting human skin and/or hair.

16. The use of claim 15, wherein anti-pollution is directed to protection and/or inhibition and/or reduction of disorders and dysfunctions of human skin and/or hair.
